# EUROPEAN PATENT SPECIFICATION

(11) **EP 1 991 289 B1**
(45) Date of publication and mention of the grant of the patent: **24.06.2015**
(21) Application number: 07752474.2
(22) Date of filing: 08.03.2007
(51) Int. Cl.: A61M 1/16, A61M 1/28

(54) **WEARABLE KIDNEY**
TRAGBARE NIERE
REIN PORTABLE

(30) Priority: 08.03.2006 US 371216
(43) Date of publication of application: 19.11.2008
(73) Proprietor: Fresenius Medical Care Holdings, Inc., Waltham, MA 02451 (US)
(72) Inventor: CURTIN, Conor, Westford, MA 01886 (US); LIPPS, Benjamin, J., Boston, MA 02210 (US); OFSTHUN, Norma, J., Lexington, MA 02421 (US); SANDFORD, Harold, F., Groton, MA 01450 (US); STENNETT, Amanda, Waltham, MA 02453 (US); UPDYKE, David, Walnut Creek, CA 94598 (US)
(74) Representative: Suèr, Steven Johannes
(86) International application number: PCT/US2007/005779
(87) International publication number: WO 2007/103411

(56) References cited:
- EP-A- 0 064 393
- WO-A-2004/009158
- DE-A1- 2 558 363
- DE-A1- 3 110 128
- US-A- 3 707 967
- US-A- 4 094 775
- US-A- 5 944 684
- US-A1- 2003 114 787
- BLUMENKRANTZ M J ET AL: "Applications of the Redy Sorbent System to Hemodialysis and Peritoneal Dialysis" ARTIFICIAL ORGANS, BLACKWELL SCIENTIFIC PUBLICATIONS, INC., BOSTON, US, vol. 3, no. 3, August 1978 (1978-08), pages 230-236, XP009088722 ISSN: 0160-564X cited in the application

## Description

### BACKGROUND OF THE INVENTION

Renal dysfunction or failure and, in particular, end-stage renal disease, causes the body to lose the ability to remove water and minerals and excrete harmful metabolites, maintain acid-base balance and control electrolyte and mineral concentrations within physiological ranges. Toxic uremic waste metabolites including urea, creatinine and uric acid accumulate in the body's tissues which can result in a person's death if the filtration function of the kidney is not replaced.

Dialysis is commonly used to replace kidney function by removing these waste toxins and excess water. In one type of dialysis treatment- hemodialysis-toxins are filtered from a patient's blood externally in a hemodialysis machine. Blood passes from the patient through a dialyzer separated by a semi-permeable membrane from a large volume of externally-supplied dialysate. The waste and toxins dialyze out of the blood through the semi-permeable membrane into the dialysate, which is then discarded. Hemodialysis treatment typically lasts several hours and must be performed under medical supervision three or four times a week, requirements that significantly decrease a patient's autonomy and quality of life. Also, since hemodialysis is performed periodically instead of continuously, the patient's condition and general well-being tend to be poor both immediately before (when toxin levels are high) and after (when electrolytes are imbalanced) hemodialysis, resulting in the patient having symptoms that range from nausea and vomiting to edema.

Peritoneal dialysis is another type of dialysis treatment used to replace kidney function in which sterile, pyrogen-free dialysis solution is infused into the patient's peritoneal cavity. The peritoneal membrane serves as a natural dialyzer and toxic uremic waste metabolites and various ions diffuse from the patient's bloodstream across the membrane into the dialysis solution via an osmotic gradient. The dialysis solution is removed, discarded and replaced with fresh dialysis solution on a semi-continuous or continuous basis. Although not all peritoneal dialysis systems require medical supervision in a treatment center, draining, discarding and replacing the large volumes of solution needed for peritoneal dialysis is still inconvenient, unwieldy and expensive.

To address this problem, devices have been designed that reconstitute used dialysate from hemodialysis and/or peritoneal dialysis as opposed to discarding it. The solution can be regenerated in a machine employing a device that eliminates urea from the solution. For example, the original Redy® (REcirculating DYalysis) Sorbent System (Blumenkrantz et al., Artif. Organs 3(3):230-236, 1978) consists of a sorbent cartridge having five layers through which dialysate solution containing uremic waste metabolites flows in order to be regenerated. The spent dialysate flows through a purification layer that removes heavy metals (i.e., copper and lead) and oxidants (i.e., chlorine and chloramine), an aluminum oxide layer containing urease bound to some of the aluminum oxide which degrades the urea in the dialysate into ammonium carbonate, a zirconium phosphate layer that adsorbs the ammonium ions produced from urea degradation along with other cations (i.e, potassium, magnesium and calcium), a hydrated zirconium oxide layer that binds phosphate and other anions (i.e., fluoride and sulfate) in exchange for acetate and an activated carbon layer that adsorbs other organic compounds (i.e., creatinine and uric acid).

Typically, the ion exchange resins used in devices such as the Redy® Sorbent System adsorb not only the urea degradation products, but also essential ions like calcium and magnesium that have diffused into the peritoneal dialysis solution. These ions must then be rapidly replaced in the patient; however, there currently exists no easy or convenient mechanism to do so. Further, although hemodialysis and peritoneal dialysis dialysate can be regenerated, no device has yet been devised that both operates continuously, clears uremic waste metabolites effectively and is small enough and/or weighs little enough to actually be comfortably worn by a patient.

There is a need for a dialysis device that is safe and effective and that significantly improves a patient's quality of life over current devices and methods. What is required is a dialysis device that operates regularly enough such that the patient does not feel unwell for significant periods of time and one that does not consume large blocks of the patient's time, require medical supervision, require volumes of dialysate so large that the patient must practically remain stationary, nor remove essential ions and minerals from the patient that then must be replaced externally. It would also be advantageous for the system to be safe enough for a patient to wear continuously and perform normal activities with little worry; that is, a system that does not involve the filtration of blood (e.g., hemodialysis), as a malfunction or disconnect within the blood circulation system can easily occur and result in rapid blood loss and death. Thus, there would be a great benefit to a dialysis system that truly allows a patient to function independently. Of further benefit would be a peritoneal dialysis device that is capable of reconstituting the dialysis solution without also removing essential ions from the patient.

US 4094775 (A) discloses a hemodialysis system which utilizes a second polymeric membrane having dialyzate in contact with one surface and a urea decomposition solution in contact with the other surface. The membrane selectively passes urea from the dialyzate into the decomposition solution, while preventing passage of positively charged metal ions from the dialyzate into the solution and ammonium ions from the solution into the dialyzate.

DE 3110128 (A1) discloses a method of dialysis in which in a first stage N-containing and/or aromatic toxins are removed and, where appropriate, urea is cleaved to ammonium bicarbonate, in a second stage the disturbed balance of the substances present as cations in the solutions to be regenerated is restored, in a third stage the disturbed balance of the substances present as anions is restored and, at the same time, the pH is made suitable for physiological requirements and the resulting regenerate is returned to the patient's blood circulation or used anew for detoxication and regeneration. There is also disclosed an apparatus which comprises a container for the adsorbents of the first stage, the cation exchanger, molecular sieves and anion exchanger with rigid or flexible boundaries, a membrane which is permeable to CO2 in the second stage, a sterilizing filter at the outlet of the regeneration unit and a pH measurement point upstream of the return of the regenerate to another blood detoxication cycle.

US 5944684 (A) discloses a wearable peritoneal dialysis device providing continuous removal of waste metabolites from the blood using a small volume (250-1000 mls) of protein-containing dialysate having an overall flow rate of about 2-3 liters per hour. The device may be worn in several places on the torso and limbs. Included are methods for peritoneal dialysis having improved efficiency, as well as methods for providing hormones, nutrients or therapeutic agents to a patient.

WO 2004009158 (A2) discloses peritoneal dialysis in which spent dialysate from the patient's peritoneal cavity passes, along a patient loop, through a dialyzer having a membrane that separates waste components from the spent dialysate, wherein the patient loop returns fresh dialysate to the patient's peritoneal cavity. The waste components are carried away in a second regeneration loop to a regeneration unit or sorbent cartridge, which absorbs the waste components. The regeneration unit removes undesirable components in the dialysate that were removed from the patient loop by the dialyzer, for example, excess water (ultra filtrate or UF), toxins and metabolic wastes. Desirable components can be added to the dialysate by the system, such as glucose and electrolytes. The additives assist in maintaining the proper osmotic gradients in the patient to perform dialysis and provide the necessary compounds to the patient.

In particular, WO 2004009158 (A2) discloses a replaceable cartridge having the features of the preamble of claim 1. The present invention is characterized by the features of the characterizing portion of claim 1. The present invention, in a further aspect, relates to a wearable peritoneal dialysis system as claimed in claim 13.

### SUMMARY OF THE INVENTION

The present invention provides a peritoneal dialysis device that can be comfortably worn by a patient continuously, 24 hours a day, 7 days a week as an alternative to conventional hemodialysis or peritoneal dialysis treatments. The dialysis device, recirculates peritoneal dialysis solution that is regenerated using a replaceable cartridge that minimizes the loss of cations from the patient. The dialysis treatment can be continuous or semi-continuous.

Accordingly, the invention relates to a wearable peritoneal dialysis system. In one embodiment, the system comprises a closed fluid system loop that circulates a volume of peritoneal dialysis solution into and out of a patient's peritoneal cavity through one or more access ports. Attached to the fluid system loop of the wearable peritoneal dialysis system are: at least one pump for moving the peritoneal dialysis solution into and out of the patient's peritoneal cavity, a replaceable drain container for removing excess fluid resulting from osmosis of the fluid from the patient's body into the peritoneal dialysis solution, a filter for removing particulates and debris from the peritoneal dialysis solution and a replaceable cartridge for regenerating the peritoneal dialysis solution attached to the fluid system loop, the replaceable cartridge having an urea removal layer that is comprised of a composition that rejects calcium and magnesium cations so that the cations are retained in the peritoneal dialysis solution and thus, in the patient.

In one embodiment, the wearable peritoneal dialysis system can further comprise a mix container attached to the fluid system loop to re-mix the regenerated peritoneal dialysis solution with an additional osmotic agent, as needed, to achieve the required peritoneal osmotic flows. In yet another embodiment, the wearable peritoneal dialysis device further comprises a filter attached to the fluid system loop that removes bacterial contamination from the regenerated peritoneal dialysis solution. A microprocessor can be in communication with the fluid system loop components to control the pump flow rates and the timing and sequencing of the components of the dialysis system, wherein the microprocessor can also be designed to be controlled externally as well.

The invention also relates to a replaceable cartridge that regenerates peritoneal dialysis fluid, the replaceable cartridge comprising a purification layer, a urea removal layer that rejects cations and an ion exchange layer. The purification layer removes heavy metals, oxidants and other uremic waste metabolites from the peritoneal dialysis fluid and, in one embodiment, is comprised of activated carbon. The ion exchange layer removes phosphate and sulfate from the peritoneal dialysis fluid and can consist of a polymeric phosphate binder or an inorganic sorbent.

The urea removal layer is comprised of a composition that repels cations yet allows urea to pass through. Thus, urea is removed from the patient but essential ions including calcium and magnesium are retained in the patient and other cations like sodium and potassium are prevented from accumulating in the replaceable cartridge, extending the life of the cartridge. In one embodiment, the composition that rejects cations is hollow fibers comprised of an ion-selective nanofiltration membrane, hollow fibers containing a layer of material that rejects cations, an ion-exchange membrane or an encapsulation surrounding the urea removal components, the encapsulation comprised of a material that rejects cations. The ion-rejecting material comprising the cation-rejecting composition or the encapsulant can be materials that reject cations by electrostatic repulsion, hydrophobicity, size exclusion, partitioning or a combination of the foregoing.

In addition to a composition that rejects cations, in one embodiment, the urea removal layer is also comprised of a composition that removes urea from the peritoneal dialysis solution. In one embodiment, the urea removal layer comprises a strong acid cation exchange resin that adsorbs the urea, together with a basic resin. In another embodiment, the urea removal layer is further comprised of an urea-degrading enzyme and at least one ion exchange sorbent that adsorbs the urea degradation products. In one embodiment, the urea-degrading enzyme is urease and, in another embodiment, the urease is attached to resin beads or the wall of hollow or solid fibers.

A method of removing uremic waste metabolites from a patient using the wearable peritoneal dialysis system of the present invention comprises pumping a volume of peritoneal dialysis solution through one or more access ports into a patient's peritoneal cavity such that the patient's uremic waste metabolites diffuse across the peritoneal membrane into the peritoneal dialysis solution; pumping the dialysis solution containing uremic waste metabolites out of the patient and into the system; draining excess fluid into a replaceable drain container; filtering particulates and debris from the peritoneal dialysis solution; regenerating the peritoneal dialysis solution containing uremic waste metabolites using a replaceable cartridge containing an urea removal layer that comprises a. composition that rejects cations arid returning the regenerated peritoneal dialysis solution to the patient's peritoneal cavity.

Unlike dialysis systems to date, the wearable peritoneal dialysis system of the present invention provides for a dialysis device that can allow the patient to maintain a relatively normal, active lifestyle. Hence, the wearable peritoneal dialysis system is far safer than wearable hemodialysis and/or filtration systems which have a very real risk of line break or disconnect from the patient which can lead to rapid and fatal blood loss. Due to the regeneration of the peritoneal dialysis solution, a relatively a small volume of solution needs to be circulated in the wearable peritoneal dialysis system which allows the system to be small and lightweight and thus, comfortable to wear overall. As the wearable peritoneal dialysis system is able to operate continuously through regeneration of the peritoneal dialysis solution, it dramatically improves a patient's overall well-being and quality of life, freeing the patient from dialysis systems that are labor-intensive, time-consuming and/or require medical supervision for operation. Regeneration of the peritoneal dialysis solution in a continuously wearable peritoneal dialysis system also means that patients do not have to frequently connect and disconnect fluid connectors to the peritoneum, a requirement in conventional peritoneal dialysis that frequently introduces infection at the connection site. Moreover, the wearable peritoneal dialysis system regenerates the peritoneal dialysis solution without removing essential ions from the solution and, ultimately, from the patient's body. This is most advantageous as, currently, these essential ions can not be replaced in the patient as quickly or effectively as necessary.

### BRIEF DESCRIPTION OF THE DRAWINGS

FIG. 1 is a schematic illustrating the fluid system loop of a wearable peritoneal dialysis system according to the invention.
FIG. 2 is a schematic illustrating a modified fluid system loop of the wearable peritoneal dialysis system according to the invention.
FIG. 3 is a drawing illustrating a replaceable cartridge of a wearable peritoneal dialysis system according to the invention.
FIG. 4 is a drawing illustrating a hollow fiber device in the urea removal layer of a replaceable cartridge containing a strong acid cation exchange adsorbent and a basic resin.
FIG. 5 is a drawing illustrating a hollow fiber in a replaceable cartridge that has a coating that repels cations.
FIG. 6 is a drawing illustrating a hollow fiber device in a replaceable cartridge that contains urease to degrade urea and a sorbent to adsorb the ammonium produced by urea degradation.
FIG. 7 is a table outlining specifications for a fluid system loop including a replaceable cartridge of the wearable peritoneal dialysis system.

### DETAILED DESCRIPTION OF THE INVENTION

The present invention generally relates to a continuous, wearable peritoneal dialysis system that removes uremic waste metabolites from a patient suffering from a disorder associated with the accumulation of uremic toxins (e.g., chronic kidney failure). The system can be used to treat a disorder like, renal disease, for example, including early renal disease, renal dysfunction or renal failure (e.g., end stage renal disease). As used herein, the term "uremic waste metabolites" refers to compounds, such as those containing nitrogen, produced by the body as waste products and includes compounds like urea, uric acid, creatinine and β2-microglobulin and other materials (see Vanholder R. et al., Kidney International 63:1934-1943, 2003). Renal failure or dysfunction leads to uremic toxicity which occurs when the levels of uremic waste metabolites in a patient are elevated compared to the levels of the toxins in individuals with normal renal function.

Thus, the present invention relates to a wearable peritoneal dialysis system that, unlike previous systems and devices, can be small enough in size to be wearable without significant burden to a patient 24 hours a day, 7 days a week. The peritoneal dialysis can be performed continuously or semi-continuousiy as the system contains a replaceable cartridge that regenerates the peritoneal dialysis solution that is then re-circulated in the system. The wearable peritoneal dialysis system is envisioned to be relatively small in size, for example, 500 to 1000 cubic centimeters (cc) in total volume. Alternatively, the components of the peritoneal dialysis system can also be assembled as a small or portable home use device. In this case, each component of the device may be larger or manufactured in such a way that it is useful as an in-home therapy (e.g. NxStage® or Allient® system).

The wearable peritoneal dialysis system is comprised of one or more access ports coupled to a component to provide inflow to and outflow from the patient's peritoneal cavity, where the component can include medically appropriate plastic tubing, a double lumen catheter or two single lumen catheters. The wearable peritoneal dialysis system also contains a volume of peritoneal dialysis solution that is infused into and out of the patient's peritoneal cavity such that the peritoneal dialysis solution removes uremic waste metabolites that diffuse through the peritoneal membrane into the peritoneal dialysis solution. Preferably, the system continuously re-circulates the peritoneal dialysis solution for maximum mass transport of the uremic toxins across the peritoneal membrane, although periodic dwell times could be advantageous for fluid removal. Any peritoneal dialysis solution can be used (e.g., Delflex), these solutions being commercially available (e.g., Fresenius Medical Care North America) and well-known in the art. A volume of about 0.5 to 3 liters of peritoneal dialysis solution can be introduced into the wearable peritoneal dialysis system and it is preferable that about 2 liters of the solution be infused. The peritoneal dialysis solution can also comprise a material added to the solution that binds uremic toxins attached to proteins in the serum. For example, albumin can be added to the peritoneal dialysis solution in the removal of these protein-bound toxins.

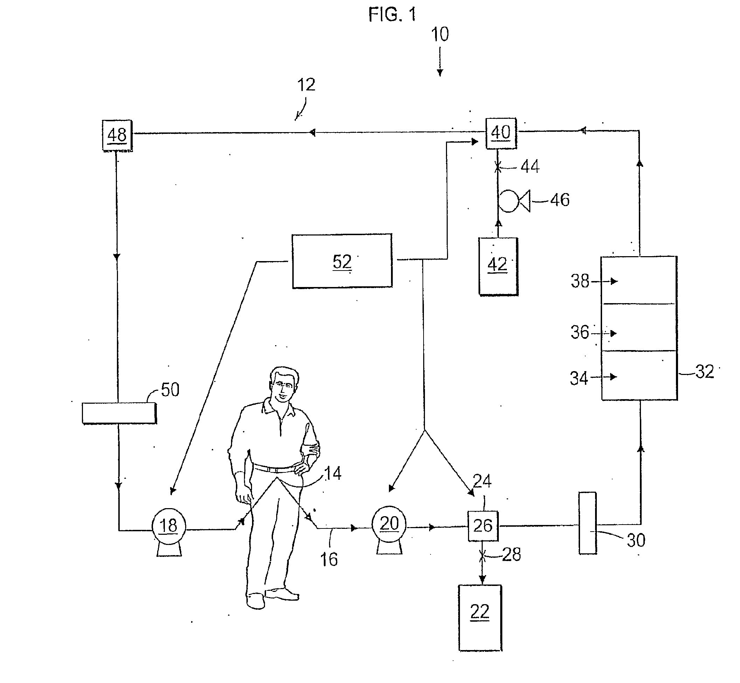

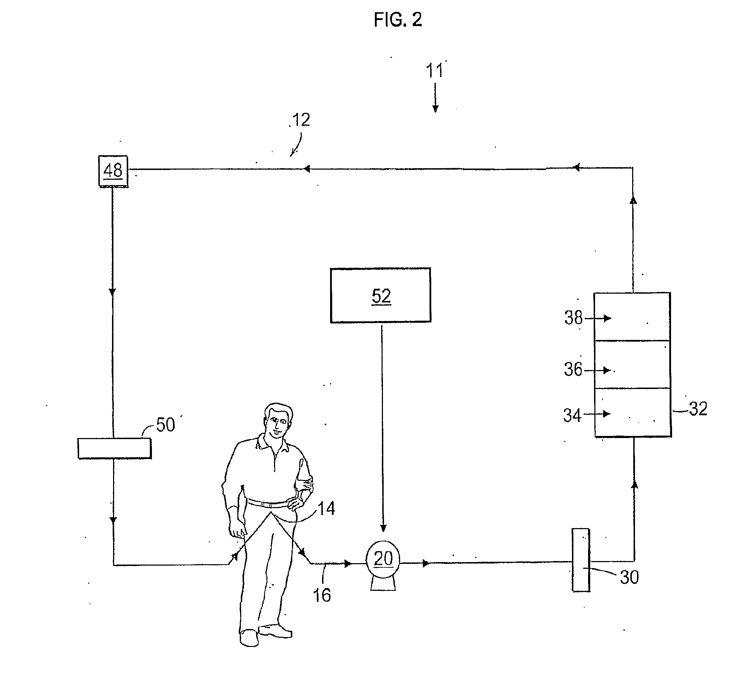

Turning to FIG. 1, a wearable peritoneal dialysis system 10 is comprised of a closed, fluid system loop 12 that circulates the peritoneal dialysis solution from the patient through access port 14, throughout the components of the fluid system loop 12 along fluid path 16 and back to the patient. In one embodiment, there is at least one pump attached to the fluid system loop to both infuse peritoneal dialysis solution into the patient's peritoneal cavity and move the peritoneal dialysis solution containing uremic waste metabolites out of the peritoneal cavity and into the fluid system loop 12. There can be at least one such pump in the fluid system loop to aid in the circulation of the peritoneal dialysis solution. As shown in FIG. 1, the peritoneal dialysis solution is infused into the patient via inflow pump 18 and the peritoneal dialysis solution, containing uremic waste metabolites and other ions that have diffused into the peritoneal dialysis solution through the peritoneal membrane, is moved out of the patient via out-flow pump 20. The one or more pumps can be any small and/or miniature pumps known in the art (e.g., Harvard Shuttle Pump). In one embodiment, the peritoneal dialysis solution is pumped through the fluid loop system at a rate of about 50 to 500 milliliters/minute (mL/min). In another embodiment, the peritoneal dialysis solution is moved through the system with one pump (e.g., pump 20) (see wearable peritoneal dialysis system 11 in FIG. 2).

Also attached to fluid system loop 12 is a replaceable drain container 22 which drains excess fluid 24 that has been added to the peritoneal dialysis solution through osmosis from the patient's body. The wearable peritoneal dialysis system 10 can be further comprised of a three-way valve 26 attached to the fluid system loop 12 that is an outlet to the replaceable drain container 22 and an on-off switch 28 (between the three-way valve 26 and the replaceable drain container 22) which regulates the drainage of excess fluid 24. The drainage of the excess fluid (ultrafiltration) can occur at a rate as determined to be appropriate by the skilled artisan and preferably at a rate of about 0.5 to 2 liters per 24 hour period. The drainage of excess fluid can occur periodically with dialysis being continuous, where the patient periodically empties the excess fluid from the replaceable drain container. Alternatively, the dialysis can be performed for a specified period of time and the drainage of excess fluid can occur during a period of time subsequent to the dialysis. For example, the dialysis can be performed for 20 hours of the day and ultrafiltration for 4 hours of the day. Alternatively, dialysis can be performed 12 hours of the day with ultrafiltration occurring 4 hours of the day, leaving the peritoneal cavity free of peritoneal dialysis solution (i.e., "dry") for 8 hours of the day. Allowing the peritoneal cavity to remain dry for several hours of the day reportedly can extend the functional lifetime of the patient's peritoneal membrane. Thus, in this and other embodiments having shorter dialysis periods (e.g., 2 hours), a drain container is not required (see FIG. 2).

The wearable peritoneal dialysis system 10 can also be comprised of a filter 30 attached to the fluid system loop 12 that removes particulates, debris and, if desired, some proteins from the peritoneal dialysis solution containing uremic waste metabolites. Numerous filters of the appropriate size and molecular weight cut off (MWCO) can be used and are commercially available (e.g., Millipore). Filter 30 can be comprised of any effective membranous material, and typically would be made up of materials like cellulose, nylon, polyvinylidene fluoride, polysulfone, polyethersulfone and polypropylene: Preferably, filter 30 would be easily replaceable and/or disposable such that the filter could be changed when saturated with particulates and/or debris, for example.. In one embodiment of the invention, the filter is no larger in diameter than the replaceable cartridge, such that it can be worn, and has a MWCO of about 100 kDa.

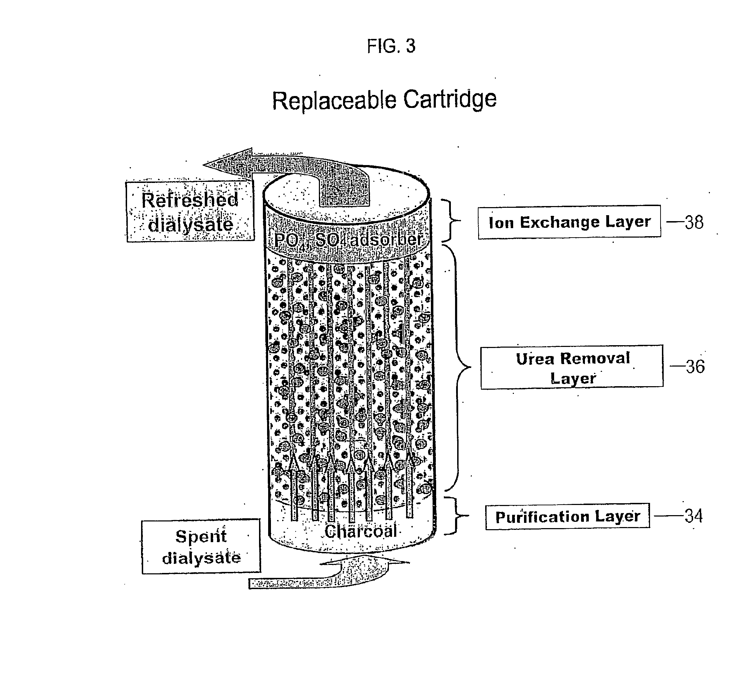

The peritoneal dialysis solution which is circulated through fluid system loop 12 continuously, is regenerated by a replaceable cartridge 32 attached to the fluid system loop. The replaceable cartridge is made up of three principal sections: a purification layer 34 that removes heavy metals, oxidants and other uremic waste metabolites from the peritoneal dialysis solution, a urea removal layer 36 that eliminates urea from the solution but rejects positively charged ions (e.g., sodium, potassium, calcium, magnesium) so that the cations are retained in the solution and an ion exchange layer 38 that removes phosphate and sulfate from the peritoneal dialysis solution (see also FIG. 3). The components of the replaceable cartridge of the invention are reduced in size compared to existing devices in order to allow the device to be easily worn on the patient's body. To be wearable, it is preferable that the dimensions of the replaceable cartridge be as small as possible to be the least obtrusive. Advantageously, the cartridge and its components can be replaced, thus when the contents of the various layers become saturated by the particular agents each layer binds and/or eliminates, the layer/section of the cartridge and/or the entire cartridge itself can be removed and easily replaced. Moreover, the sections of the device can be sterilized and/or regenerated for re-use.

Accordingly, in the replaceable cartridge, the peritoneal dialysis solution first flows through purification layer 34 which typically is comprised of activated carbon/charcoal. The solution next flows through urea removal layer 36 which is made up of urea removal components and a composition that rejects cations. As used herein, the term "urea removal components" refers to components of the replaceable cartridge that eliminate urea by adsorbing (e.g., via a strong acid cation exchange resin) or breaking down (e.g., via an urea-degrading enzyme) the urea and binding and/or removing (e.g., using a strong acid cation exchange resin or ion exchange sorbent) the byproducts of the urea elimination reactions. Urea removal layer 36 is also comprised of a composition able to reject cations that have diffused from the patient into the peritoneal dialysis solution in the patient's peritoneal cavity via a concentration gradient. The cation-rejecting composition is comprised of ion-selective elements that prevent cations from being removed from the peritoneal dialysis solution and includes hollow fibers made of an ion-selective nanofiltration membrane.

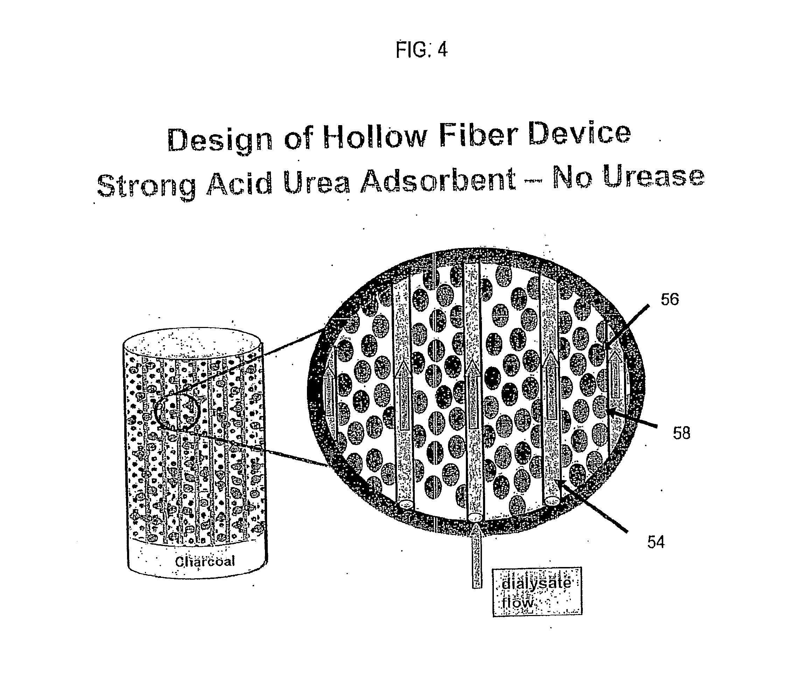

Thus, in one embodiment, the urea removal layer is made up of a strong acid cation exchange resin (e.g., styrene/divinylbenzene sulfonic acid cation exchange resin) and a basic (alkaline) anion exchange resin (e.g., Dowex 1(OH)) or a dual-property resin (e.g., Bio-Rad AG 51-X8) to remove urea (see also FIG. 4). As used herein, the term "dual-property resin" refers to an ion exchange resin that can act as both a strong acid cation exchange resin and a basic (alkaline) anion exchange resin. In addition to the strong acid and basic resin(s), the urea removal layer can also be comprised of hollow fibers 54 made of an ion-selective nanofiltration membrane (available from, e.g., Amerida, Koch, GE, Hoechst and Dow) that prevents cation diffusion from the peritoneal dialysis solution. Alternatively, in another embodiment, the ion-rejecting component can be an ion-selective encapsulation (e.g., cellulose acetate) that surrounds the strong and basic resins or the dual-property resin, the encapsulation allowing the urea through but repelling cations. In yet another embodiment, the urea removal layer can be comprised of a urea-degradation enzyme (e.g., urease) and an ion exchange resin (e.g., strong acid cation exchange) or inorganic sorbent (e.g., zirconium phosphate), the enzyme and sorbent encapsulated with a cation-rejecting material (e.g., cellulose acetate). The composition that rejects cations can be comprised of hollow fibers made of an ion-selective material. The material covering the hollow fibers or surrounding the urea removal components would most likely be either positively charged or relatively impermeable to polar molecules, causing it to reject cations.

To complete the regeneration of the peritoneal dialysis solution, the solution then flows through ion exchange layer 38 that removes phosphate and sulfate from the peritoneal dialysis solution. The ion exchange layer can be comprised of either a polymeric phosphate binder (e.g., Renagel®) or an ion exchange sorbent (e.g., hydrous zirconium oxide). The replaceable cartridge of the wearable peritoneal dialysis system preferably removes phosphate from the patient at a rate of about 8 to 12 milliliters/minute (mL/min) and clears urea from the patient at a rate of about 10 to 30 mL/min. For the removal of 20 g of urea in 24 hours, the urea would be cleared at a rate of 10 to 15 mL/min whereas removal of 20 g of urea in 12 hours would require a urea removal rate of 20 to 30 mL/min. Sulfate is preferably cleared from the patient at a rate of about 50 milliequivalents (mEq) per 24 hours and, similarly, hydrogen ions are cleared from the patient at a rate of about 60 to 70 mEq in a 24 hour period. The regeneration of the peritoneal dialysis solution in the replaceable cartridge, which is recirculated in the wearable peritoneal dialysis system, allows a small volume of the solution to be used in the system such that it is light and compact enough to be worn by a patient with ease.

The wearable peritoneal dialysis system 10 can be further comprised of mix container 42 attached to fluid system loop 12 so that an osmotic agent (e.g., glucose, glucose polymer, amino acids) can be added, as necessary, to maintain the correct osmotic induced flow in the peritoneum. Accordingly, the wearable peritoneal dialysis system can be further comprised of a three-way valve 40 attached to the fluid system loop 12 that serves as an outlet to the mix container 42; an on-off flow switch 44 between the three-way valve 40 and the mix container 42 that regulates flow of the regenerated peritoneal dialysis solution into the container; and a flow pump 46 between the on-off switch 44 and the mix container 42 that contains a solution comprising an osmotic agent, the pump serving to infuse the osmotic agent solution into the mix container with the regenerated peritoneal dialysis solution. In one embodiment, the osmotic agent is glucose which is added to achieve or maintain a concentration of up to about 4.25 percent. In addition, the wearable peritoneal dialysis system can contain a three-way valve 48 that connects the flow of the re-mixed and regenerated peritoneal dialysis solution to an initial priming point of the fluid system loop. These components, however, are not required and, in embodiments in which.the dialysis period is short and/or semi-continuous, the mix container can be eliminated (see FIG. 2).

A filter 50 able to remove bacterial contamination from the regenerated peritoneal dialysis solution can also be attached to the fluid system loop 12 of the wearable dialysis system. Filters that remove and/or eliminate bacteria are known in the art and are commercially available (e.g., JMC, A-M Systems, Millipore and Direct Med., Inc). The filter can be comprised of any material (e.g., cellulose, polyethersulfone, nylon, polyester or polystyrene) appropriate to exclude and/or sequester bacteria from the solution based on size and/or chemical or biological properties of the bacteria and would only need to be of the correct shape and size to fit appropriately in the wearable peritoneal dialysis system. Thus, the filter diameter is envisioned to be no larger than the replaceable cartridge and have a filtration cut-off of about 0.1 microns or less. Bacterial filter 50 would, preferably, also be removable, regenerable and/or replaceable.

As a means of controlling the components of the wearable peritoneal dialysis system, in one embodiment of the invention microprocessor 52 can be in communication with the components of the system (e.g., inflow pump 18, out flow pump 20, three-way valve 26 and/or three way valve 40). Microprocessor 52 can control, alter and adjust the pump flow rates and the timing and sequencing of the components of the dialysis system in response to pre-programmed instructions or according to the patient's needs as determined by the skilled clinician. The wearable peritoneal dialysis system 10 could also contain sensors able to measure uremic toxin concentrations such that microprocessor 52 can calculate relevant biostatistics (e.g., level of uremic waste metabolites removed or ions adsorbed) and be programmed to adjust accordingly the pump speed, for example, such that the patient receives the most efficacious treatment. Microprocessor 52 is preferably located within the unit housing the wearable peritoneal dialysis system 10 itself to direct and coordinate the components of the dialysis system. There could also be an external, wireless control system (e.g., another microprocessor) that could, as needed; direct and adjust the wearable peritoneal dialysis system through the microprocessor 52 that is within the wearable dialysis system unit itself.

The wearable peritoneal dialysis system can also be used in conjunction with a source of one or more enzymes capable of degrading uremic waste metabolites as described in O'Loughlin et al., Tissue Eng. 10:1446-1455, 2004 and O'Loughlin et al. US2005/0123529.

O' Loughlin *et al.* discloses methods to reduce the concentration of uremic toxins *in vivo* by either orally delivering to a patient with renal dysfunction enzymes, generally encapsulated, or organisms and/or cells capable of eliminating and/or degrading uremic toxins. A patient can orally ingest encapsulated enzymes that are able to degrade uremic waste metabolites, the metabolites degraded by the enzymes in the gastrointestinal tact. Oral administration of the enzymes in conjunction with the use of the peritoneal dialysis system decreases the load of uremic waste metabolites needing to be removed from the patient by the wearable peritoneal dialysis system, allowing the system to contain a smaller urea removal component for regenerating the dialysis solution and, consequently, be more easily worn. Further, the orally ingested enzymes, by breaking down the uremic waste metabolites, allow the smaller degradation products to be more easily removed by the wearable peritoneal dialysis system and/or the patient's intestines. The source of enzymes can include enzymes known to degrade uremic waste metabolites like uricase, urease or creatininase, or any other suitable enzymes known to one having skill in the art, or a cell naturally occurring or genetically engineered that degrades uremic waste metabolites through the expression of one or more degradation enzymes or proteins that regulate the one or more enzymes' expression or activity.

The enzymes can be administered by any suitable method including direct administration of the enzymes (e.g., as a pharmaceutical composition in an appropriate carrier), in an encapsulation (e.g., a capsule, sustained release pill or liposome) or direct administration of a cell that expresses the enzymes (e.g., a microbial, yeast or mammalian cell in a suitable carrier). In a particular embodiment, the enzymes can be encapsulated in a material like silicone, polystyrene, alginate, other polymers, cellulose, any combination of the aforementioned materials or any other medically appropriate, non-toxic material known to those of skill in the art. The encapsulation surrounding the sorbent and/or enzymes can also reject cations such that these ions are not adsorbed by the sorbent and are not removed from the patient's body. A single enzyme can be encapsulated or one or more enzymes can be encapsulated provided that the one or more enzymes are able to break down urea. The degraded uremic waste metabolites can be delivered to and eliminated by the intestines. The enzymes can be administered with a sorbent (i.e., an ion exchange sorbent like zirconium phosphate) that can adsorb the urea degradation products. In a preferred embodiment, the sorbent is encapsulated with one or more enzymes, and, in another embodiment, is in a separate encapsulation from the one or more enzymes. Generally, the sorbent would also be orally administered. If the uremic waste metabolites are degraded by a cell (e.g., a microbe), the cell itself may sequester the degradation products, which are then eliminated from the patient's body with the cell.

The amount of enzymes or cells administered to a patient to sufficiently decrease the load of uremic waste metabolites can be determined by one with skill in the art and will vary from patient to patient. The dosage will depend on the severity of the renal failure or dysfunction, the patient's age, body weight, overall well-being and the particular agent chosen under the specific conditions of administration. Preferably, the dosage does not have a negative effect on the patient. The source of the one or more enzymes can be administered once or several times during a 24 hour period, the schedule of administration dependent on the patient's need to meet a particular level of clearance of uremic waste metabolites and the patient's tolerance as determined by the skilled clinician and based on experimental models and clinical results.

The present invention further relates to a replaceable cartridge for use in the system that regenerates the peritoneal dialysis fluid in the system without adsorbing excessive amounts of cations (e.g., calcium, magnesium, sodium, potassium) that, through a concentration gradient, have diffused from the patient's body into the peritoneal dialysis solution in the peritoneum. The replaceable cartridge for use in the wearable peritoneal dialysis system contains a purification layer, urea removal layer that rejects cations in the peritoneal dialysis solution and an ion exchange layer. The cartridge and/or its components or layers can be replaced (e.g., membrane, urea-degrading enzyme), regenerated (e.g., resin, sorbent) and/or sterilized for re-use when necessary (e.g., saturation, damage, depletion). In addition, the entire cartridge can be replaceable and thus removed from the wearable peritoneal dialysis system when there is a decrease in the regeneration efficiency of the cartridge (e.g., through layer saturation) or the cartridge becomes worn or damaged, for instance. As seen in FIG. 3, peritoneal dialysis solution enters the replaceable cartridge, first encountering purification layer 34 which, like the purification layer of the device of the Redy® URS System (Renal Solutions, Inc.), removes heavy metals (e.g., lead, mercury, arsenic, cadmium, chromium and thallium), oxidants (e.g., chlorine and chloramine) and other uremic waste - metabolites (e.g., creatinine and uric acid) using activated carbon, typically charcoal. Preferably, the activated carbon would have a large surface area per volume, a wide range of pore sizes for adsorbing various size uremic toxins and a high purity and/or USP grade. High purity of the carbon may be achieved through multiple acid and/or water washes to remove any water soluble impurities. It would also be advantageous for the carbon to be in a granular or pressed form in order to limit the pump power required. Examples of appropriate activated carbon include: Nuchar Aquaguard 40, Norit ROX, and Norit E Supra.

The peritoneal dialysis solution next flows through urea removal layer 36 which can, in a number of ways, eliminate urea from the solution while allowing positively charged ions and, in some cases, essential ions to be retained in it. In one embodiment, the layer is comprised of a strong acid cation exchange resin, a strong base anion resin and a composition that rejects cations. The strong acid and basic resins can be separate resins, or one dual-property mixed bead resin. Strong acid cation resins are well-known in the art (e.g., Amberlyst™ 36, 131, 15, 31, 35, 39, 40 and 70; DOWEX™ C, C-10, C-350, C-400, 650C(H), 575C NG(H), N406, G-26(H), HCR-S/S, HCR-W2, HGR-W2, MSC, 88, M-31, MP-525C(H), DR-2030, MC-575(H), MSC-1, 88 MB and 88; Rexyn™ resins) and are commercially available (e.g., Rohm and Haas, Dow and Fisher-Scientific). Positive counter ions (e.g., hydrogen and/or sodium) may be released through the process of ion exchange in the strong acid cation resin. The released hydrogen ions are bound by a basic (alkaline) resin, to maintain the pH of the peritoneal dialysis solution in the desired (e.g., physiological) range. The basic (alkaline) resin can be any appropriate polyamine ion (e.g., anion) exchange resin available or its acid salt complex including: DOWEX 66, 77, WBA, WBA-2, WB-500, M-43, XUS 43594.00, and XUS 43568.00, Amberlite IRA67, IRA743, IRA96 and others, these resins available from Dow and Rohm and Haas, for example. As shown in FIG. 4, the strong acid and basic resins are distinct and the composition that rejects positively charged ions are hollow fibers, the hollow fibers comprised of an ion-selective nanofiltration membrane. The peritoneal dialysis solution travels through hollow fibers 54, the urea passing through hollow fibers 54 and adsorbed by strong acid cation resin 56. Basic ion exchange resin 58 helps to maintain the appropriate pH (e.g., physiological) of the solution as described above. By rejecting cations, the hollow fibers allow these ions to be retained in the peritoneal dialysis solution that is returned to the patient. Advantageously, as urea is not broken down, urea degradation products (e.g., ammonium carbonate) are not formed and thus, do not have to also be removed from the peritoneal dialysis solution.

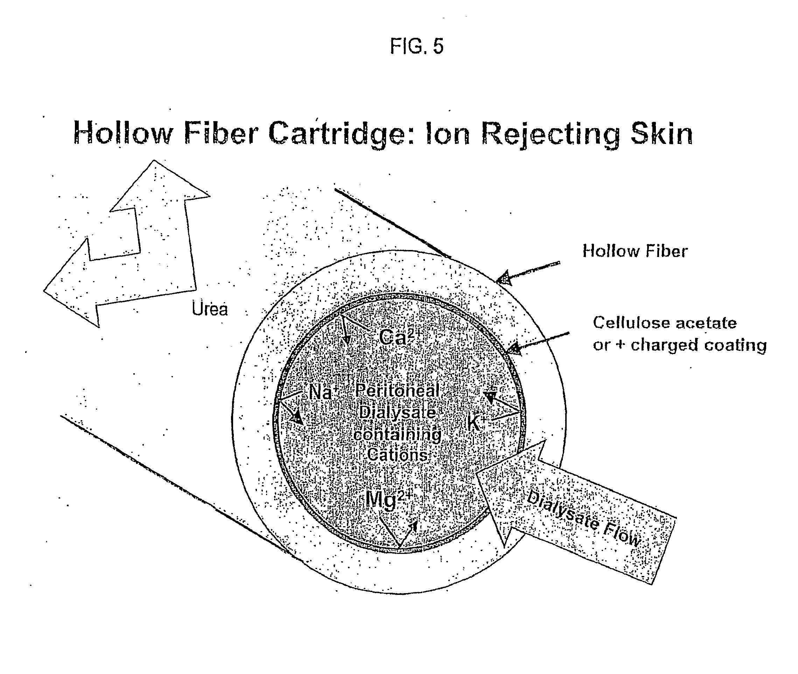

An embodiment in which hollow fibers are fabricated from or coated with an ion-rejecting material is depicted in FIG. 5. A layer can be formed on the hollow fibers by coating or co-extruding them with a material which allows the urea through but rejects positively charged ions. The material covering the hollow fibers can be any known to one of skill in the art (e.g., fatty acids or polymer chains like cellulose acetate) that can effectively reject cations and therefore retain the ions in the peritoneal dialysis solution. Alternatively, the material can be positively charged; that is, the material can have a multitude of positively charged groups (e.g., quarternary ammonium groups) attached to a polymer film which is either coextruded with the hollow fiber material or coated on the fibers after fabrication. In one embodiment, the material used to cover the hollow fibers is cellulose acetate, in particular, cellulose diacetate and/or cellulose triacetate. Hollow fibers are commercially available (e.g., Fresenius Medical Care North America) and, for use in the invention, need only be able to be covered with the desired cation-rejecting material. Alternatively, the hollow fibers can be comprised of an ion-selective nanofiltration membrane, similar to those commercially available from a number of sources (e.g., Amerida, Koch, GE, Hoechst and Dow). These membranes have pores sizes that prevent ionic substances from diffusing through the membrane. For example, there are nanofiltration membranes that have an ability to reject ions with more than one positive charge (e.g., calcium, magnesium) while allowing single-charged ions (e.g., sodium) to pass through. In either case, the hollow fiber devices are available in a variety of dimensions and need only be small enough to fit in the replaceable cartridge, which can be sized to be comfortably worn or sized for use in an in-home system.

In another example nor forming part of the invention, cation-rejecting composition can be a flat membrane that is covered with a positively charged material like those described above. In addition, the membrane can be an ion exchange (e.g., anion).membrane that limits the .passage of positively charged ions (e.g., Astrom® Neosepta® AFX anion exchange membrane, PCA GmbH PC-SA anion exchange membrane). Advantageously, this ion exchange membrane also has an ability to adsorb phosphate, reducing the need for/level of phosphate-removing compositions in the ion-exchange layer of the replaceable cartridge.

In yet another example not forming part of the invention, the strong acid and basic (alkaline) resins or dual-property resin (e.g., mixed bed) can themselves be encapsulated by a material through which urea can pass, but cations can not. Hence, the peritoneal dialysis solution flows into the urea removal layer comprised of the encapsulated resin(s) and the urea in the peritoneal dialysis solution diffuses through the encapsulation where it is adsorbed by the strong acid or dual-property resin. In a particular embodiment, the strong acid cation exchange resin is a sulfonic acid based resin in the protonated hydrogen (H+) form. The positive counter ions produced are adsorbed by the basic ion exchange resin also present in the encapsulation or by the dual-property resin. Cations in the peritoneal dialysis solution are prevented from passing through the ion-rejecting encapsulation. The encapsulation can be comprised of the materials previously discussed that would reject cations by electrostatic repulsion (e.g., positively charged polymers), hydrophobicity (e.g., fatty acids), size exclusion (e.g., nanofiltration), partitioning (e.g., cellulose acetate) or a combination of the foregoing properties.

Urea can also be removed from the peritoneal dialysis solution using one or more enzymes that degrade urea. Thus, in another embodiment, the urea removal layer is comprised of an enzyme that degrades urea, an ion exchange sorbent that adsorbs the urea degradation products and a composition that rejects cations, specifically, sodium, potassium, calcium and magnesium. The enzyme can be any known to one of skill in the art that can break down urea into its ionic components (e.g., ammonium and carbonate ions). Enzymes with the correct specificity and activity that can be employed are those naturally occurring (e.g., urease from jack beans, other seeds or bacteria), produced by recombinant technology (e.g., in bacterial, fungal, insect or mammalian cells that express and/or secrete urea-degrading enzymes) or produced synthetically (e.g., synthesized). In one embodiment, the enzyme is urease... In a particular embodiment, the urease is used in conjunction with a strong acid ion exchange resin (e.g., sorbent). In this embodiment, both the urease and the strong acid resin are preferably thoroughly washed of impurities/undesirable species before use in the urea removal layer of the replaceable cartridge. Both the urease and the strong acid cation exchange resin can be washed in, for example, deionized water to remove these impurities. In particular, the strong acid resin is washed to remove contaminating acidic species (e.g., free sulfonic or sulfuric acid and low molecular weight oligomeric residues of the strong acid cation exchange resin) that remain from the manufacturing process of the resin. Removal of these acidic species prevents their leaching out during regeneration of the peritoneal dialysis solution and their resultant inactivation of urease. In addition, peptide fragments or other positively charged impurities (e.g., cationic buffer species) are preferably removed from the urease by washing so that no impurities remain that may be adsorbed by the strong acid cation exchange resin, resulting in a release of hydrogen ions that decrease the pH of the environment that inactivates the urease.

The enzyme (e.g., urease) may also be chemically attached to the membrane or, alternatively, to porous beads or a resin. This both stabilizes the enzyme for extended use and, in the case of the porous beads or resin, allows the urease to be filled and/or replaced in the device. In particular, urease may be chemically attached to the exterior of the polysulfone hollow fiber membrane or to separate fibers or resins. Attachment is through reactive pendant groups of amino acid portions of the enzyme such as thiol groups, amino groups, or carboxylic acid groups that will not affect the catalytic site. Chemistries that can be used to immobilize enzymes or cross-linked enzyme crystals (CLECs) are well-known in the art (see e.g., J.Jegan Roy and T. Emilia Abraham, Strategies in Making Cross-Linked Enzyme Crystals, Chemical Reviews, 104(9):3705-3721). In addition, urease can be used in its crystallized form and be mixed with the ion exchange resin or sorbent, for example, for degradation of the urea.

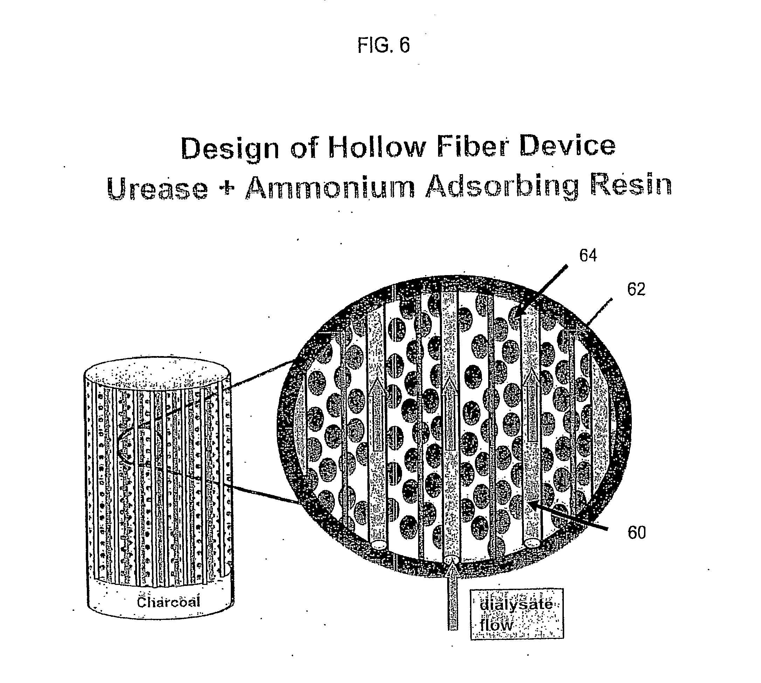

In the embodiment involving the use of urea-degradation enzymes, the composition that rejects cations is hollow fibers comprised of an ion-selective nanofiltration membrane as described above. Alternatively, but not forming part of the invention, the cations can be rejected by an encapsulation surrounding the urea degrading enzyme and an ion exchange sorbent or resin. In the embodiment shown in FIG. 6, peritoneal dialysis solution containing urea flows through hollow fibers 60. Urea passes through hollow fibers 60, where encapsulated enzymes 62 break down the urea into ammonium and carbonate, the urea degradation byproducts absorbed by ion exchange sorbent 64. The sorbent (e.g., a cation exchange resin) adsorbs the ammonium ions or free ammonia. In a preferred embodiment, the ion exchange sorbent is a strong acid cation exchange resin in the protonic form, but can be any ion exchange sorbent (e.g., zirconium phosphate) that can effectively adsorb urea degradation products. As in the previous embodiment with the strong acid and basic (alkaline) resins, hollow fibers 60 allow the urea in the peritoneal dialysis solution to diffuse through and reject positively charged ions in the solution. If the urea-degrading enzyme and ion exchange sorbent(s) are surrounded by an ion-selective encapsulation (as opposed to the urea removal layer containing hollow fibers), the urea in the peritoneal dialysis solution diffuses through the encapsulation, where it is degraded by the enzyme, and those degradation products are then bound by the ion-exchange sorbent. The ion-selective encapsulation rejects the cations in the peritoneal dialysis solution, so that they are retained in the solution. The ion-rejecting material coating the hollow fibers or comprising the encapsulation surrounding the enzyme and ion exchange resin would typically do so by electrostatic repulsion, hydrophobicity, size exclusion, partitioning or a combination of the aforementioned factors.

The replaceable cartridge is further comprised of ion exchange layer 38 (see FIGs. 1 and 2), which is designed to remove phosphate and sulfate from the peritoneal dialysis fluid after urea removal. The ion exchange layer can be comprised of those ion exchange resins able to remove phosphate and/or sulfate, for example, a strong base anion exchange resin and other applicable forms of the resin such as carbonate, bicarbonate or chloride. These resins are known to the skilled artisan who can determine the most favorable resin for use in the invention based on a number of factors, including the patient's condition and the physiological advantages of using a particular resin and the potential toxicity of the resin. For instance, the ion exchange resin can be a polymeric/polyamine phosphate binder like sevelamer hydrochloride (i.e., Renagel®, Genzyme, Inc.), poly(allylamine) and/or poly(allylamine hydrochloride). Other commercially available ion exchange resins useful for binding phosphate include: DOWEX M-43 (anion exchange resin), DOWEX 21 K XLT, DOWEX 1 (OH), DOWEX Marathon MSA and DOWEX M4195 (in the copper form). Alternatively, the ion exchange layer can be comprised of an anion exchange resin that would bind phosphate and sulfate (e.g., Amberlite™ 96, Rohm and Haas) and, in a particular embodiment, is hydrous zirconium oxide (e.g., zirconium oxide in the acetate counter ion form combined with zirconium carbonate).

Thus, after flowing through the replaceable cartridge of the invention, the peritoneal dialysis solution is essentially regenerated for reuse. The solution is largely free of urea, uric acid and creatinine, and has lower levels of phosphate and sulfate. Due to the design of the urea removal layer such that its components reject particular ions, the peritoneal dialysis solution retains sufficient levels of calcium and magnesium ions in the patient, eliminating the need for a mechanism to replace these ions in the patient. In addition, repelling cations like sodium and potassium prevents the ions from entering the replaceable cartridge, decreasing the load of ions bound to the cartridge components (e.g., the strong acid cation exchange resin of the urea removal layer) and the frequency at which the components need to be replaced/regenerated. Thus, the rejection of sodium and potassium increases the longevity of components of the replaceable cartridge and/or that of the replaceable cartridge itself.

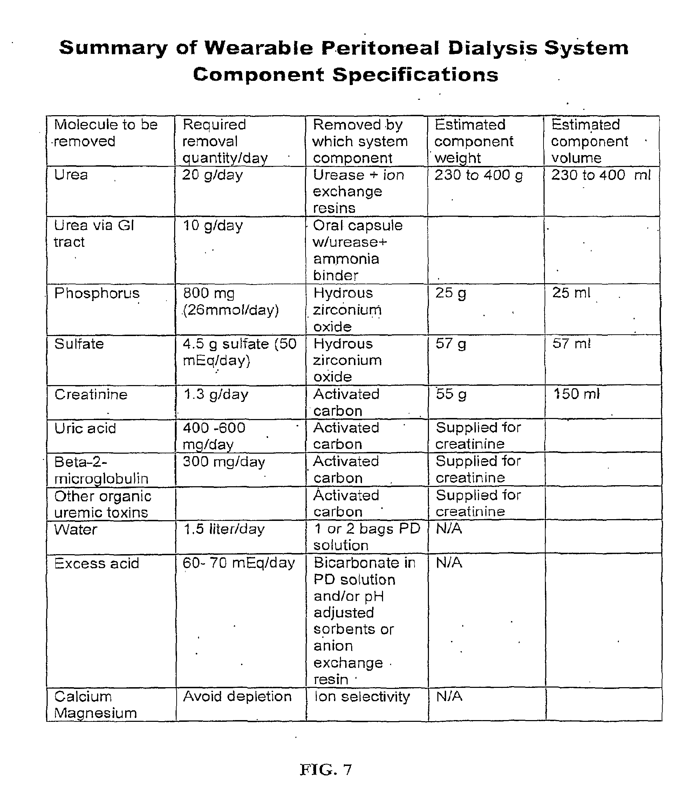

FIG. 7 presents an example of uremic toxins and the amount of various materials calculated to be necessary to remove the uremic toxins. In general, the metabolism of most dialysis patients produces 20 g of urea on a daily basis. In an embodiment in which a patient is treated with dialysis over a 12 hour period of time, hydrolysis of 20 g of urea requires at least 1000 international units (IU) of urease (1 mg). This calculation regarding the amount of urease to be used for a particular time period of dialysis is dependent on several factors including, for example, the patient's metabolism and urea levels, the purity of the urease and the activity of the urease throughout the course of the treatment and the determination of the level of urease to use in treatment of a given patient best done by the skilled artisan. The hydrolysis of 20 g of urea by the urease generates approximately 11.4 g of ammonia. It is necessary to remove this ammonia with, for example, an ion exchange resin, in this case with 230 g of a high capacity strong acid cation exchange resin or with 1200 g of zirconium phosphate. More of the strong acid cation exchange resin can be used in the instance that the resin is exposed to other cations. To maintain a neutral pH of the solution, the acid produced from the strong acid cation exchange resin and the patient themselves must be neutralized. Generally, an alkaline anion exchange resin is utilized to neutralize the acid and, as shown in FIG. 7, 70 g of the resin is used. Inclusion of sodium bicarbonate at levels best determined by one of skill in the art can help reduce the amount of anion exchange resin required to neutralize the acid/achieve neutral pH.

Excess phosphorous (phosphate) and sulfate are released from protein catabolism and food digestion. In people with normal kidney function, any excess phosphorous and sulfate are excreted by the kidneys; however, patients with kidney disease/renal insufficiency may have up to 800 mg of excess phosphorous and/or 4.5 g of excess sulfate. In the specifications shown in FIG. 7, approximately 25 g of hydrous zirconium is used to bind the estimated 800 mg phosphorous (2.4 g phosphate) and 57 g of additional hydrous zirconium oxide used to bind the 4.5 g of sulfate.

A significant number of other uremic toxins, for example, creatinine can also be removed in dialysis. In this embodiment, 55 g of high activity (activated) high surface area carbon is used to bind 1.3 g of creatinine. This activated carbon can also remove uric acid (400 to 600 mg), β-2 microglobulin (up to 300 mg) and other uremic toxins.

In the replaceable cartridge, one of skill in the art can choose the appropriate component/materials described previously to be utilized in the urea removal layer that allows for the diffusion of urea, but excludes the passage of cationic species (e.g., calcium, magnesium) across the membrane. This design of the replaceable cartridge protects the cation exchange resin from exposure to cations which reduces the release of hydrogen ions helping to prevent changes in pH. Thus, only the amount of cation exchange resin necessary to bind the ammonia present is required. The component/material also eliminates and/or reduces the loss of these cations from the patient and the resultant need to replace them fairly quickly.

The access port(s) through which the peritoneal dialysis solution is added and removed can be at a convenient and appropriate place in the patient's peritoneal cavity and can be connected to the wearable peritoneal dialysis system by any appropriate medical tubing, a double lumen catheter or a single lumen catheter. The volume of peritoneal dialysis solution initially provided in the wearable peritoneal dialysis system can be anywhere from 0.5 to 3 liters, or whatever volume deemed to be suitable to effectively clear uremic waste metabolites from the patient by one with skill in the art. The peritoneal dialysis solution is.pumped through the dialysis system at a rate of about 50 to 500 mL/min and the dialysis can occur continuously or semi-continuously. In a particular method, drainage of excess fluid from the patient occurs at a rate of about 0.5 to 3 liters per 24 hour period. If the wearable dialysis system operates continuously, as in one embodiment of the invention, the drainage of excess fluid can also be continuous, with excess fluid being periodically removed from the replaceable drain container by the patient. Alternatively, the dialysis system can operate semi-continuously for a specific period of time (e.g., 12 to 20 hours) and the removal of excess fluid takes place during a period of time subsequent to the dialysis (e.g., 4 hours). Preferably, some fresh dialysis fluid is added to the wearable kidney system once a day at a convenient time.

The peritoneal dialysis solution provided is regenerated by a replaceable cartridge having a urea removal layer that rejects cations. As before, regeneration of the peritoneal dialysis solution can decrease the amount of solution necessary to perform the dialysis and, accordingly, the size of the wearable peritoneal dialysis system. The replaceable cartridge is as described previously and regenerates the peritoneal dialysis solution through the use of a series of layers in the device, one which removes heavy metals, oxidants and other uremic waste metabolites from the solution in a purification layer, another eliminating urea from the solution without removing essential ions in a urea removal layer and yet another removing phosphate and sulfate from the peritoneal dialysis solution in an ion exchange layer. The components of the.replaceable cartridge that perform these functions are also those described previously, that is, activated carbon (in the purification layer), a polymeric phosphate binder or an ion exchange sorbent (in the ion exchange layer) and urea removal components (e.g., strong acid cation exchange resin and basic (alkaline) resin(s) or urea-degrading enzymes and an ion exchange sorbent) together with a composition that rejects cations (e.g., flat membrane/hollow fibers containing a layer of a cation-rejecting composition, flat membrane/hollow fibers comprised of an ion-selective nanofiltration membrane, an ion-exchange membrane or an encapsulation surrounding the urea removal components) (in the urea removal layer). In a preferred embodiment, the cation-rejecting layer of the flat membrane or hollow fibers or surrounding the resins and/or enzymes is positively charged, containing a substituent such as quarternary ammonium group, or the material is cellulose diacetate or cellulose triacetate, fatty acids or other appropriate polymers.

In addition, the method can further comprise orally administering to a patient a source of one or more enzymes capable of degrading uremic waste metabolites, enzymes like uricase, urease or creatininase. In doing so, the load of uremic waste metabolites that need to be removed from the patient by the wearable peritoneal dialysis system can be significantly reduced in amount or altered for ease of removal and/or intestinal elimination. The source of the orally administered enzymes can be the one or more enzymes themselves in an acceptable pharmaceutical carrier and/or in a suitable encapsulation, or naturally occurring or genetically engineered cells that can degrade uremic waste metabolites as described previously. Preferably, the enzymes together with the sorbent are administered in an encapsulated form and in some cases, this encapsulation can also reject calcium and magnesium ions. The amount and/or dosage of the source of uremic toxin-degrading enzymes administered to the patient can be appropriately determined by one with skill in the art, and is dependent on the formulation chosen, the assessed necessity to clear a particular amount of uremic waste metabolites from the patient and the patient's specifications (e.g., age, body weight and overall well-being).

The method preferably results in urea being cleared from the patient at a rate of about 10 to 30 mL/min and phosphate being cleared from the patient at a rate of about 8 to 12 mL/min. Sulfate is preferably cleared from the patient at a rate of about 50 mEq per 24 hours and hydrogen ions are preferably cleared from the patient at a rate of about 60 to 70 mEq in a 24 hour period.

In yet another method, an appropriate osmotic agent is added to the regenerated peritoneal dialysis solution in a mix container to ensure proper osmotic induced flow into the patient's peritoneal cavity. Accordingly, the method further comprises infusing a concentrated osmotic agent solution into the mix container via a flow pump between the on-off switch and the mix container, the pump regulating the flow of the regenerated peritoneal dialysis solution into the mix container; mixing the regenerated peritoneal dialysis solution with the osmotic agent solution in the mix container; and pumping the re-mixed and regenerated peritoneal dialysis solution back into the dialysis system.

In a further method, the re-mixed and regenerated peritoneal dialysis solution is filtered to remove bacterial contamination from the solution. In yet another embodiment, the re-mixed and regenerated peritoneal dialysis solution flows through a three-way valve into an initial priming point of the dialysis system before the peritoneal dialysis solution is returned to the patient's peritoneal cavity.

To consistently and efficiently remove uremic waste metabolites from a patient; control of the wearable peritoneal dialysis system and, in particular, the pump flow rates and the timing and sequencing of the components of the dialysis system are electrically controlled. In a preferred embodiment, the control mechanism is a microprocessor which is part of a unit containing the dialysis system that is under its own control; however, the microprocessor can also be controlled wirelessly, typically by another microprocessor.

### EXAMPLE

A GE Sepa^{™} lab scale crossflow membrane filtration unit was modified to enable the testing of membranes in a countercurrent diffusion mode. The unit was equipped with a Neosepta AFX-A0100 membrane. Peritoneal dialysis solution (1000 ml) spiked with 1.5 grams of urea was pumped across one side of the membrane. Deionized water (1000 ml) was circulated through the other side of the membrane and through a FMC-NA F6 dialysis cartridge (in which the hollow fibers were infused with a solution of washed urease). The deionized water was also pumped through 3 small cartridges containing ion exchange resins (two filled with Dowex 1 (OH) and one filled with a high capacity strong acid ion exchange resin from Rohm and Haas). It was found necessary to thoroughly wash the strong acid cation exchange resin, or material that leached from it deactivated the urease. Samples were removed periodically from both fluid loops and analyzed for calcium, magnesium, glucose, BUN, pH, and ammonia.

The analyses indicated that a significant portion of the urea diffused through the membrane and that there was minimal diffusion of the calcium, magnesium or sodium through the membrane. The urea that diffused through the membrane was hydrolyzed by the urease in the dialyzer hollow fibers to ammonia, which was in turn bound by the strong acid ion exchange resin. The combination of ion exchange resins maintained the pH of the solutions within a range in which the urease remained active over a period of 24 hours.

### PD Circuit

| Time (hr) | BUN (mg/dl) | Na (meq/L) | Mg (mg/dl) | Ca (mg/dl) | pH |
|---|---|---|---|---|---|
| 0.0 | 60.8 | 125 | 1.5 | 4.7 | 5.2 |
| 21.0 | 40.1 | 121 | 1.4 | 4.7 | 5.0 |
| 46.2 | 23.9 | 113 | 1.4 | 4.5 | 5.0 |

### RO Circuit

| Time (hr) | BUN (mg/dl) | NH3 (µg/dl) | Na (meq/L) | Mg (mg/dl) | Ca (mg/dl) | pH |
|---|---|---|---|---|---|---|
| 0.0 | 0.0 | 0 | 0.0 | 0.0 | 0.0 | 6.4 |
| 21.0 | 0.0 | 0 | 0.0 | 0.1 | 0.0 | 6.3 |
| 46.2 | 5.1 | 30 | 0.0 | 0.0 | 0.0 | 6.0 |

While this invention has been particularly shown and described with references to preferred embodiments thereof, it will be understood by those skilled in the art that various changes in form and details may be made therein without departing from the scope of the invention encompassed by the appended claims.

## Claims

1. A replaceable cartridge (32) for regenerating a peritoneal dialysis solution comprising:
a purification layer (34) for removing heavy metals, oxidants and other uremic waste metabolites from said peritoneal dialysis solution; and
an ion exchange sorbent layer (38) for removing phosphate and sulfate from said peritoneal dialysis solution; and
a urea removal layer (36) that rejects cations, the cartridge **characterized in that** the urea removal layer comprises hollow fibers containing a layer of material comprised of an ion-selective nanofiltration membrane that prevents cations from being removed from the peritoneal dialysis solution; and **in that**
the components of the cartridge are reduced in size relative to existing devices so as to enable the cartridge to be worn on a user's body.

2. The replaceable cartridge (32) of Claim 1 wherein the purification layer (34) comprises activated carbon.

3. The replaceable cartridge (32) of claim 1 wherein the ion exchange layer (38) is comprised of a material selected from the group consisting of a polymeric phosphate binder, an ion-exchange sorbent and a strong base anion exchange resin.

4. The replaceable cartridge (32) of claim 1 wherein the urea removal layer (36) further comprises:
a strong acid cation exchange resin to adsorb the urea; and
a basic resin to adsorb the counter ions released through ion exchange by said strong acid cation exchange resin.

5. The replaceable cartridge (32) or claim 4 wherein the strong acid cation exchange resin is washed to remove acidic species.

6. The replaceable cartridge (32) of claim 4 wherein a dual-property resin is both a strong acid cation exchange resin and a basic resin.

7. The replaceable cartridge (32) of claim 1 wherein the urea removal layer (36) further comprises:
an enzyme to degrade the urea; and
at least one ion exchange sorbent to adsorb the urea degradation products.

8. The replaceable cartridge (32) of claim 7 wherein the enzyme to degrade the urea is urease.

9. The replaceable cartridge (32) of claim 8 wherein the urease is attached to a material selected from the group consisting of resin beads, the wall of hollow fibers (60) and the wall of solid fibers.

10. The replaceable cartridge (32) of claim 8 wherein the urease is washed to remove positively charged species or peptide fragments.

11. The replaceable cartridge (32) of claim 7 wherein the ion exchange sorbent is selected from the group consisting of a zirconium phosphate sorbent and a high capacity strong acid cation exchange resin.

12. The replaceable cartridge (32) of claim 1 wherein the layers of the cartridge are one or more selected from the group consisting of removable, regenerable and replaceable.

13. A wearable peritoneal dialysis system (10) for a patient comprising:
one or more access ports (14) coupled to a component for providing inflow to and outflow from the patient's peritoneal cavity;
a volume of peritoneal dialysis solution that is infused into and moved out of the patient's peritoneal cavity, thereby removing from the patient uremic waste metabolites that have diffused into the peritoneal dialysis solution;
a closed fluid system loop (12) for circulating the peritoneal dialysis solution from the patient) throughout the system and back into the patient;
at least one pump (20) attached to the fluid system loop (12) for infusing the peritoneal dialysis solution into the patient's peritoneal cavity and moving the peritoneal dialysis solution containing uremic waste metabolites out of the patient's peritoneal cavity and into the fluid system loop (12);
a replaceable drain container (22) attached to the fluid system loop (12) for draining excess fluid (24);
a filter (30) attached to the fluid system loop (12) for removing particulates and debris from the peritoneal dialysis solution containing uremic waste metabolites; and
a replaceable cartridge (32) attached to the fluid system loop (12) for regenerating the peritoneal dialysis solution as claimed in any preceding claim.

14. The wearable peritoneal dialysis system (10) of claim 13 wherein the urea removal layer (36) further comprises urea removal components selected from the group consisting of: a strong acid cation exchange resin together with a basic resin, a dual property resin and a urea-degradation enzyme together with at least one ion exchange sorbent.

15. The wearable peritoneal dialysis system (10) of claim 14 wherein the layers of the cartridge are one or more selected from the group consisting of removable, regenerable and replaceable.

16. The wearable peritoneal dialysis system (10) of claim 15 further comprising a mix container attached to the fluid system loop (12) to re-mix the regenerated peritoneal dialysis solution with an osmotic agent.

17. The wearable peritoneal dialysis system (10) of claim 16 further comprising a three-way valve (26) to connect the flow of the re-mixed and regenerated peritoneal dialysis solution to an initial priming point of the fluid system loop (12).

18. The wearable peritoneal dialysis system (10) of claim 17 further comprising a filter (30) attached to the fluid system loop (12) to remove bacterial contamination from the regenerated peritoneal dialysis solution.

19. The wearable peritoneal dialysis system (10) of claim 18 further comprising a microprocessor in communication with the components of the fluid system loop (12), said microprocessor controlling the pump flow rates and the timing and sequencing of the components of the dialysis system.

## Patentansprüche

1. Austauschbare Patrone (32) zum Regenerieren einer Peritonealdialyselösung, die umfasst:
eine Reinigungsschicht (34) zum Entfernen von Schwermetallen, Oxidationsmitteln und anderen urämischen Abfallmetaboliten aus der Peritonealdialyselösung; und
eine Ionenaustauschsorbensschicht (38) zum Entfernen von Phosphat und Sulfat aus der Peritonealdialyselösung; und
eine Harnstoffentfernungsschicht (36), die Kationen abstößt,
wobei die Patrone **dadurch gekennzeichnet ist, dass** die Harnstoffentfernungsschicht Hohlfasern umfasst, die eine Schicht aus einem Material enthalten, das aus einer ionenselektiven Nanofiltrationsmembran besteht, die verhindert, dass Kationen aus der Peritonealdialyselösung entfernt werden; und dass
die Komponenten der Patrone eine in Bezug auf bestehende Einrichtungen verringerte Größe aufweisen, so dass möglich wird, dass die Patrone am Körper eines Benutzers getragen wird.

2. Austauschbare Patrone (32) nach Anspruch 1, wobei die Reinigungsschicht (34) Aktivkohle umfasst.

3. Austauschbare Patrone (32) nach Anspruch 1, wobei die Ionenaustauschschicht (38) aus einem Material besteht, das aus der Gruppe ausgewählt ist, bestehend aus einem Polymerphosphatbindemittel, einem Ionenaustauschsorbens und einem Anionenaustauschharz starker Base.

4. Austauschbare Patrone (32) nach Anspruch 1, wobei die Harnstoffentfernungsschicht (36) darüber hinaus umfasst:
ein Kationenausstauschharz starker Säure, um den Harnstoff zu adsorbieren; und
ein basisches Harz, um die Gegenionen zu adsorbieren, die durch einen Ionenaustausch durch das Kationenaustauschharz starker Säure freigesetzt werden.

5. Austauschbare Patrone (32) oder Anspruch 4, wobei das Kationenaustauschharz starker Base gewaschen wird, um saure Spezies zu entfernen.

6. Austauschbare Patrone (32) nach Anspruch 4, wobei ein Harz mit zweifacher Eigenschaft sowohl ein Kationenaustauschharz starker Säure als auch ein basisches Harz ist.

7. Austauschbare Patrone (32) nach Anspruch 1, wobei die Harnstoffentfernungsschicht (36) darüber hinaus umfasst:
ein Enzym, um den Harnstoff abzubauen; und
zumindest ein Ionenaustauschsorbens, um die Harnstoffabbauprodukte zu adsorbieren.

8. Austauschbare Patrone (32) nach Anspruch 7, wobei das Enzym zum Abbauen des Harnstoffs Urease ist.

9. Austauschbare Patrone (32) nach Anspruch 8, wobei die Urease an ein Material angehaftet ist, das aus der Gruppe ausgewählt ist, bestehend aus Harzkügelchen, der Wand aus Hohlfasern (60) und der Wand aus Massivfasern.

10. Austauschbare Patrone (32) nach Anspruch 8, wobei die Urease gewaschen wird, um positiv geladene Spezies oder Peptidfragmente zu entfernen.

11. Austauschbare Patrone (32) nach Anspruch 7, wobei das Ionenaustauschsorbens aus der Gruppe ausgewählt ist, bestehend aus einem Zirkoniumphosphatsorbens und einem hochkapazitiven Kationenaustauschharz starker Säure.

12. Austauschbare Patrone (32) nach Anspruch 1, wobei die Schichten der Patrone eines oder mehreres ausgewählt aus der Gruppe, bestehend aus entfernbar, regenerierbar und austauschbar, sind.

13. Tragbares Peritonealdialysesystem (10) für einen Patienten, das umfasst:
eine oder mehrere Zugangsöffnungen (14), die mit einer Komponente verbunden sind, um einen Zufluss in die Peritonealhöhle des Patienten und einen Ausfluss aus dieser heraus bereitzustellen;
ein Volumen einer Peritonealdialyselösung, das in die Peritonealhöhle des Patienten infundiert und aus dieser heraus bewegt wird, wodurch urämische Abfallmetaboliten aus dem Patienten entfernt werden, die in die Peritonealdialyselösung diffundiert sind;
einen geschlossenen Fluidsystemkreis (12) zum Zirkulieren der Peritonealdialyselösung aus dem Patienten durch das gesamte System und zurück in den Patienten;
zumindest eine Pumpe (20), die an den Fluidsystemkreis (12) angeschlossen ist, um die Peritonealdialyselösung in die Periotonealhöhle des Patienten zu infundieren und die Peritonealdialyselösung, die die urämischen Abfallmetaboliten enthält, aus der Peritonealhöhle des Patienten heraus und in den Fluidsystemkreis (12) zu bewegen;
einen austauschbaren Abflussbehälter (22), der an dem Fluidsystemkreis (12) angeschlossen ist, um überschüssiges Fluid (24) abzufließen;
einen Filter (30), der an dem Fluidsystemkreis (12) angeschlossen ist, um Partikel und Ablagerungen aus der Peritonealdialyselösung zu entfernen, die urämische Abfallmetaboliten enthält; und
eine austauschbare Patrone (32), die an dem Fluidsystemkreis (12) angeschlossen ist, um die Peritonealdialyselösung zu regenerieren, nach einem vorstehenden Anspruch.

14. Tragbares Peritonealdialysesystem (10) nach Anspruch 13, wobei die Harnstoffentfernungsschicht (36) darüber hinaus Harnstoffentfernungskomponenten umfasst, die aus der Gruppe ausgewählt sind, bestehend aus: einem Kationenaustauschharz starker Säure gemeinsam mit einem basischen Harz, einem Harz mit zweifacher Eigenschaft und einem Harnabbauenzym gemeinsam mit zumindest einem Ionenaustauschsorbens.

15. Tragbares Peritonealdialysesystem (10) nach Anspruch 14, wobei die Schichten der Patrone eines oder mehreres ausgewählt aus der Gruppe, bestehend aus entfernbar, regenerierbar und austauschbar, sind.

16. Tragbares Peritonealdialysesystem (10) nach Anspruch 15, das darüber hinaus einen Mischbehälter umfasst, der an dem Fluidsystemkreis (12) angeschlossen ist, um die regenerierte Peritonealdialyselösung neu mit einem Osmosemittel zu vermischen.

17. Tragbares Peritonealdialysesystem (10) nach Anspruch 16, das darüber hinaus ein 3-Wege-Ventil (26) umfasst, um den Strom der neu vermischten und regenerierten Peritonealdialyselösung mit einem ersten Priming-Punkt des Fluidsystemkreises (12) zu verbinden.

18. Tragbares Peritonealdialysesystem (10) nach Anspruch 17, das darüber hinaus einen Filter (30) umfasst, der an dem Fluidsystemkreis (12) angeschlossen ist, um eine bakterielle Kontaminierung aus der regenerierten Peritonealdialyselösung zu entfernen.

19. Tragbares Peritonealdialysesystem (10) nach Anspruch 18, das darüber hinaus einen Mikroprozessor umfasst, der mit den Komponenten des Fluidsystemkreises (12) in Kommunikationsverbindung steht, wobei der Mikroprozessor die Pumpdurchflussraten und den Zeitpunkt sowie die Sequenzierung der Komponenten des Dialysesystems steuert.

## Revendications

1. Cartouche remplaçable (32) pour la régénération d'une solution de dialyse péritonéale, comprenant :
une couche de purification (34) destinée à éliminer les métaux lourds, les oxydants et d'autres métabolites de déchets urémiques dans ladite solution de dialyse péritonéale ; et
une couche d'adsorbant à échange d'ions (38) destinée à éliminer le phosphate et le sulfate dans ladite solution de dialyse péritonéale ; et
une couche d'élimination d'urée (36) rejetant des cations,
la cartouche étant **caractérisée en ce que** la couche d'élimination d'urée comprend des fibres creuses contenant une couche de matériau constitué d'une membrane de nanofiltration sélective d'ions, empêchant l'élimination des cations dans la solution de dialyse péritonéale ; et **en ce que**
les composants de la cartouche sont réduits en taille par rapport à des dispositifs existants, de manière à permettre le port de la cartouche sur le corps d'un utilisateur.

2. Cartouche remplaçable (32) selon la revendication 1, dans laquelle la couche de purification (34) comprend du charbon activé.

3. Cartouche remplaçable (32) selon la revendication 1, dans laquelle la couche d'échange ionique (38) est constituée d'un matériau sélectionné parmi le groupe comprenant un liant de phosphate polymérique, un adsorbant à échange d'ions et une résine échangeuse d'anions à base forte.

4. Cartouche remplaçable (32) selon la revendication 1, dans laquelle la couche d'élimination d'urée (36) comprend en outre :
une résine échangeuse de cations fortement acides pour l'adsorption de l'urée ; et
une résine basique pour l'adsorption des contre-ions libérés par échange d'ions par ladite résine échangeuse de cations fortement acides.

5. Cartouche remplaçable (32) selon la revendication 4, dans laquelle la résine échangeuse de cations fortement acides est lavée pour éliminer des espèces acides.

6. Cartouche remplaçable (32) selon la revendication 4, dans laquelle une résine à double propriété est à la fois une résine échangeuse de cations fortement acides et une résine basique.

7. Cartouche remplaçable (32) selon la revendication 1, dans laquelle la couche d'élimination d'urée (36) comprend en outre :
une enzyme destinée à dégrader l'urée ; et
au moins un adsorbant à échange d'ions pour l'adsorption des produits de dégradation de l'urée.

8. Cartouche remplaçable (32) selon la revendication 7, dans laquelle l'enzyme destinée à dégrader l'urée est l'uréase.

9. Cartouche remplaçable (32) selon la revendication 8, dans laquelle l'uréase est fixée sur un matériau sélectionné parmi le groupe comprenant des perles de résine, la paroi de fibres creuses (60) et la paroi de fibres solides.

10. Cartouche remplaçable (32) selon la revendication 8, dans laquelle l'uréase est lavée pour éliminer des espèces ou fragments de peptide chargés positivement.

11. Cartouche remplaçable (32) selon la revendication 7, dans laquelle l'adsorbant à échange d'ions est sélectionné parmi le groupe comprenant un adsorbant de phosphate de zirconium et une résine échangeuse de cations fortement acides à grande capacité.

12. Cartouche remplaçable (32) selon la revendication 1, dans laquelle les couches de la cartouche sont l'une ou plusieurs sélectionnées parmi le groupe comprenant une couche amovible, une couche régénérable et une couche remplaçable.

13. Système de dialyse péritonéale ambulatoire (10) destiné à un patient, comprenant :
un ou plusieurs orifices d'accès (14) accouplés à un composant pour fournir un flux entrant dans la cavité péritonéale du patient et un flux sortant de celle-ci ;
un volume de solution de dialyse péritonéale administré dans la cavité péritonéale du patient et extrait de celle-ci, éliminant ainsi chez le patient les métabolites de déchets urémiques diffusés dans la solution de dialyse péritonéale ;
un circuit de système de fluide fermé (12) pour la circulation de la solution de dialyse péritonéale, depuis le patient, à travers tout le système et de nouveau vers le patient ;
au moins une pompe (20) reliée au circuit de système de fluide (12) pour l'administration de la solution de dialyse péritonéale dans la cavité péritonéale du patient et pour l'évacuation de la solution de dialyse péritonéale contenant des métabolites de déchets urémiques hors de la cavité péritonéale du patient et dans le circuit de système de fluide (12) ;
un récipient de drainage remplaçable (22) relié au circuit de système de fluide (12) pour le drainage du fluide en excès (24) ;
un filtre (30) relié au circuit de système de fluide (12) pour l'élimination de particules et de débris dans la solution de dialyse péritonéale contenant des métabolites de déchets urémiques ; et
une cartouche remplaçable (32) reliée au circuit de système de fluide (12) pour la régénération de la solution de dialyse péritonéale selon l'une quelconque des revendications précédentes.

14. Système de dialyse péritonéale ambulatoire (10) selon la revendication 13, dans lequel la couche d'élimination d'urée (36) comprend en outre des composants d'élimination d'urée sélectionnés parmi le groupe comprenant : une résine échangeuse de cations fortement acides combinée à une résine basique, une résine à double propriété et une enzyme de dégradation d'urée combinée à au moins un adsorbant à échange d'ions.

15. Système de dialyse péritonéale ambulatoire (10) selon la revendication 14, dans lequel les couches de la cartouche sont l'une ou plusieurs des couches sélectionnées dans le groupe comprenant une couche amovible, une couche régénérable et une couche remplaçable.

16. Système de dialyse péritonéale ambulatoire (10) selon la revendication 15, comprenant en outre un récipient de mélange relié au circuit de système de fluide (12) et servant à remélanger la solution de dialyse péritonéale régénérée avec un agent osmotique.

17. Système de dialyse péritonéale ambulatoire (10) selon la revendication 16, comprenant en outre une vanne à trois voies (26) pour relier le flux de la solution de dialyse péritonéale régénérée et remélangée à un point d'amorçage initial du circuit de système de fluide (12).

18. Système de dialyse péritonéale ambulatoire (10) selon la revendication 17, comprenant en outre un filtre (30) relié au circuit de système de fluide (12) pour éliminer une contamination bactérienne de la solution de dialyse péritonéale régénérée.

19. Système de dialyse péritonéale ambulatoire (10) selon la revendication 18, comprenant en outre un microprocesseur en communication avec les composants du circuit de système de fluide (12), ledit microprocesseur contrôlant les débits de pompe et la synchronisation et le séquençage des composants du système de dialyse.
